# EUROPEAN PATENT APPLICATION

(11) **EP 4 653 328 A2**
(43) Date of publication of application: **26.11.2025**
(21) Application number: 25174307.6
(22) Date of filing: 05.05.2025
(51) Int. Cl.: B65B 3/00, A61L 2/14, B65B 55/10, A61L 2/22

(54) **DISINFECTING A PACKAGE TO BE FILLED WITH A LIQUID FOOD PRODUCT USING HYDROGEN PEROXIDE**

(30) Priority: 20.05.2024 EP 24176831
(71) Applicant: Tetra Laval Holdings & Finance S.A., 1009 Pully (CH)
(72) Inventor: Niedbal, Dominika, 221 86 Lund (SE)
(74) Representative: Tetra Pak Patent Attorneys

(57) **Abstract**

The present invention relates to a packaging machine (100) configured to fill packages with a liquid food product. The packaging machine comprising: a feed unit (110) configured to feed packaging material to be formed to packages to be filled or to feed packages to be filled; and a packaging disinfection station (120) configured to disinfect the packaging material or the packages. The packaging disinfection station comprising: a spray nozzle (124) configured to distribute an aerosol onto the packaging material or onto the packages; a hydrogen peroxide supply (122) configured to supply a liquid comprising hydrogen peroxide to the spray nozzle; and an ionizer (126) configured to ionize hydrogen peroxide of the aerosol before the aerosol is distributed onto the packaging material or onto the packages. Also a packaging disinfection station (120) configured to disinfect a package to be filled with a liquid food product is presented. Further, a method for filling a package with a liquid food product during which method disinfecting an interior of the package with hydrogen peroxide is presented.

## Description

### Technical field

The present invention relates to disinfection of packages and/or packaging material in connection with filling packages with a liquid food product. Especially, a packaging machine comprising a packaging disinfection station configured to disinfect packaging material or packages using hydrogen peroxide. A packaging disinfection station configured to disinfect packaging material or packages using hydrogen peroxide is also presented. Moreover, a method for filling a package with a liquid food product during which method the package is disinfected using hydrogen peroxide is also presented.

### Background of the invention

Hydrogen peroxide, H₂O₂, is a potent oxidizing agent, meaning it can effectively kill a wide range of microorganisms including bacteria, viruses, fungi, and spores. This broad spectrum of activity makes it a versatile disinfectant for various surfaces, including packages. Unlike some other disinfectants, hydrogen peroxide breaks down into water and oxygen after use, leaving behind no harmful residue. This makes it safe for use on surfaces that come into contact with food or other sensitive items.

Further, in order to enhance its effectiveness as a disinfectant hydrogen peroxide may be activated. Activating hydrogen peroxide make it more potent and efficient at disinfecting surfaces, including packages, by increasing its ability to kill microorganisms and break down organic contaminants. This activation typically involves applying light, typically UV-light, to initiate chemical reactions that produce more reactive oxygen species, such as hydroxyl radicals. Activation of hydrogen peroxide is a two-step process in which hydrogen peroxide first is applied to the surface to be disinfected and thereafter the hydrogen peroxide is activated using UV-light.

Such activating comes with challenges. First of all a two-step process involves challenges in that the same surface is to both being treated with hydrogen peroxide and exposed to UV-light. Moreover, generation of UV-light for industrial purposes is most often done using mercury UV-lamps. However, such lamps are currently being phased out from use.

Hence, there is a need of improvements in applying activated hydrogen peroxide for disinfecting purposes in connection with packaging machine, especially packaging machine configured to fill packages with a liquid food product.

### Summary of the invention

The herein disclosed technology seeks to at least partly mitigate, alleviate or eliminate one or more of the above-mentioned deficiencies and disadvantages in the prior art. In particular, it is an object to provide efficient disinfection of packages and/or packaging material in connection with filling packages with liquid food product.

The inventor of the present inventive concept has realized a new and improved way of disinfecting packages in connection with high speed filling of the same with liquid food product.

Various aspects and embodiments of the disclosed invention are defined below and in the accompanying independent and dependent claims.

According to a first aspect, a packaging machine configured to fill packages with a liquid food product is provided. The packaging machine is typically a roll-fed packaging machine or a blank-fed packaging machine. The packaging machine comprises a feed unit configured to feed packaging material to be formed to packages to be filled or to feed packages to be filled; and a packaging disinfection station configured to disinfect the packaging material or the packages. The packaging disinfection station comprises: a spray nozzle configured to distribute an aerosol onto the packaging material or onto the packages, a hydrogen peroxide supply configured to supply a liquid comprising hydrogen peroxide to the spray nozzle; and an ionizer configured to ionize hydrogen peroxide of the aerosol before the aerosol is distributed onto the packaging material or onto the packages.

By ionizing the hydrogen peroxide of the aerosol before the aerosol is distributed onto the packaging material or onto the packages improved disinfection may be achieved. This since it may be safeguarded that at least most of hydrogen peroxide is activated to form hydroxyl radicals. That is, the aerosol formation and the activation of the hydrogen peroxide is made in a combined one step approach as compared with the prior art in which first the hydrogen peroxide is distributed onto surfaces to be disinfected and thereafter the distributed hydrogen peroxide is activated using UV-light.

The ionizer maybe a plasma generator comprising two pin electrodes between which a plasma is configured to be formed. This provide for continuous production of ionized hydrogen peroxide.

The ionizer may be arranged in close proximity distance of 0,2 mm to 1 cm from an outlet of the spray nozzle. This provide for control of the ionization of the hydrogen peroxide. That is, it may be safeguarded so that substantially all hydrogen peroxide undergo ionization.

The packages to be filled may have a cross-section defined by a plurality of sidewalls, typically four, a closed end engaging the sidewalls, and an open end. At least the sidewalls of the packages are typically carton based packages. The spray nozzle may be configured to distribute the aerosol within an interior of the packages to be filled.

The spray nozzle may be configured to be inserted into the interior of the packages to be filled. Upon the spray nozzle is inserted into the interior of a package to be filled the spray nozzle is configured to distribute the aerosol onto interior surfaces of the package to be filled. This allow for increased control of the distribution of the ionized hydrogen peroxide.

The aerosol may comprise 3-10 vol% of hydrogen peroxide.

The spray nozzle may be configured to produce droplets of aerosol having a mean diameter of 40 nanometers to 3 micrometers.

The packaging machine may further comprise a removing station configured to remove hydrogen peroxide from the disinfected packaging material or packages before the packages are filled with the liquid food product. The removing of residue hydrogen peroxide is typically performed using 90 °C to 200 °C hot HEPA filtered air.

The packaging machine may further comprise a filling station configured to fill the packages to be filled with the liquid food product.

The packaging machine may further comprise a sealing station configured to seal packages filled with the liquid food product. Especially, the sealing station is configured to close and seal the open end of the packages.

The packaging machine may be configured to fill at least 4500 packages per hour per packaging line.

According to a second aspect a packaging disinfection station is disclosed. The packaging disinfection station is configured to be feed with packaging material to be formed to packages (20) to be filled or to be feed with packages to be filled. The packaging disinfection station is configured to disinfect packaging material of the packages to be filled with a liquid food product. The packages to be filled typically having a cross-section defined by a plurality of sidewalls, a closed end engaging the sidewalls, and an open end. The packaging disinfection station comprises: a spray nozzle configured to distribute an aerosol onto the packaging material or onto the packages feed to the packaging disinfection station; a hydrogen peroxide supply configured to supply a liquid comprising hydrogen peroxide to the spray nozzle; and an ionizer arranged in close proximity of an outlet of the spray nozzle to ionize hydrogen peroxide of the aerosol before the aerosol is distributed onto the packaging material or onto the packages.

The spray nozzle may be configured to be inserted into an interior of a package to be filled and to distribute the aerosol onto interior surfaces of the package to be filled.

The above mentioned features of the first aspect, when applicable, apply to this second aspect as well. In order to avoid undue repetition, reference is made to the above.

According to a third aspect a method for filling a package with a liquid food product is provided. The method comprises providing a package to be filled, the provided package to be filled having a cross-section defined by a plurality of sidewalls, a closed end engaging the sidewalls, and an open end and disinfecting an interior of the package to be filled. The disinfecting is made by: inserting a spray nozzle configured to distribute an aerosol comprising hydrogen peroxide into the package to be filled; distributing, via the spray nozzle, the aerosol comprising hydrogen peroxide onto inner surfaces of the package to be filled; ionizing the hydrogen peroxide of the aerosol before the aerosol is distributed onto the inner surfaces of the package to be filled; withdrawing the spray nozzle from the package to be filled; and filling the disinfected package with the liquid food product.

The method may further comprising removing hydrogen peroxide residue from the disinfected packages before the packages are filled with the liquid food product. The removing may be performed with 90 °C to 200 °C hot HEPA filtered air.

The method may further comprise sealing the filled packages.

The above mentioned features of the first or second aspects, when applicable, apply to this second aspect as well. In order to avoid undue repetition, reference is made to the above.

A further scope of applicability of the present disclosure will become apparent from the detailed description given below. However, it should be understood that the detailed description and specific examples, while indicating preferred variants of the present inventive concept, are given by way of illustration only, since various changes and modifications within the scope of the inventive concept will become apparent to those skilled in the art from this detailed description.

### Brief description of the drawings

The above and other aspects of the present inventive concept will now be described in more detail, with reference to appended drawings showing variants of the present inventive concept. The figures should not be considered limiting the invention to the specific variant; instead, they are used for explaining and understanding the inventive concept.

As illustrated in the figures, the sizes of layers and regions are exaggerated for illustrative purposes and, thus, are provided to illustrate the general structures of variants of the present inventive concept. Like reference numerals refer to like elements throughout.
Fig. 1 schematically illustrates a packaging machine configured to fill packages with a liquid food product.
Figs 2a and 2b illustrates one exemplified embodiment of a packaging disinfection station to be used in the packaging machine of Fig. 1. In Fig. 2a, a package to be disinfected is arranged in register with the packaging disinfection station. In Fig. 2b, a spray nozzle of the packaging disinfection station has been inserted into the package to be disinfected.
Fig. 3 is a block diagram of a method for filling a package with a liquid food product.

### Detailed description

The present inventive concept will now be described more fully hereinafter with reference to the accompanying drawings, in which currently preferred variants of the inventive concept are shown. This inventive concept may, however, be implemented in many different forms and should not be construed as limited to the variants set forth herein; rather, these variants are provided for thoroughness and completeness, and fully convey the scope of the present inventive concept to the skilled person.

It will also be appreciated that when the present disclosure is described in terms of a method, it may also be embodied in an apparatus or device comprising one or more processors, one or more memories coupled to the one or more processors, where computer code is loaded to implement the method. For example, the one or more memories may store one or more computer programs that perform the steps, services and functions disclosed herein when executed by the one or more processors.

It is also to be understood that the terminology used herein is for the purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. The term "obtaining" is herein to be interpreted broadly and encompasses receiving, retrieving, collecting, acquiring, and so forth.

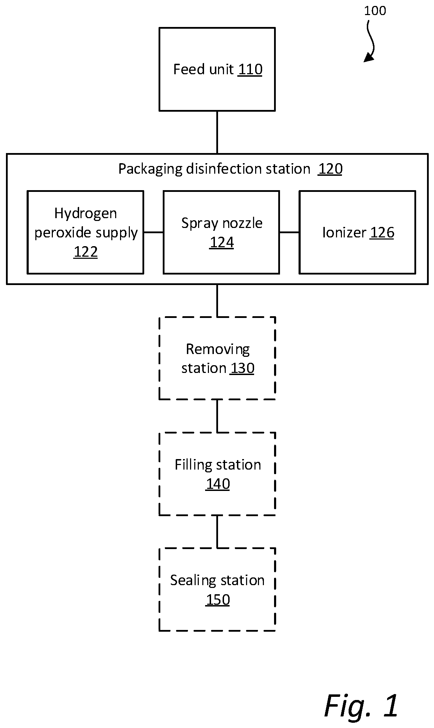

Fig. 1 generally illustrates, a packaging machine 100 configured to fill packages with a liquid food product. The packaging machine 100 may be a roll-fed packaging machine used for producing packages. That is the packaging machine 100 may be feed with a web of packaging material to be formed into packages to be filled. More specifically, the packaging machine 100 may be used for packaging liquid food products in carton-based packages. Already in the 1940s this type of packaging machines was introduced by Tetra Pak^{®} and it is today a well-known approach for packaging milk and other liquid food products in a safe and cost-efficient manner. Alternatively, the packaging machine 100 may be a blank-fed packaging machine in which the packaging material is provided as blanks to the packaging machine 100. The packaging material, regardless of if it is provided as a web of packaging material or as blanks, typically comprises a carton layer and at least one plastic layer. The packaging material is often printed and prepared in packaging material production centers, also referred to as converting factories, and shipped to a site where the packaging machine 100 is placed, e.g. a dairy. For a roll-fed packaging machine 100 the packaging material is loaded onto a reel before being transported. After arriving at the site, the reel is placed in the packaging machine 100, and a web of packaging material is fed in the packaging machine 100 by a feed unit 110. In case of the packaging machine 100 being a blank-fed packaging machine, the package material is provided as blanks to the packaging machine 100 and a feed unit 110 is feeding the blanks to downstream stations within the packaging machine 100.

The packaging machine 100 comprises the above discussed feed unit 110 configured to feed packaging material within the packaging machine 100 to be formed to packages to be filled. The feed unit 110 may further be configured to feed the packages formed within the packaging machine 100 to other downstream stations within the packaging machine 100. Hence, the feed unit 110 may comprise a plurality of sub feed units.

The packaging machine 100 further comprises a packaging disinfection station 120. The packaging disinfection station 120 is configured to disinfect the packaging material, in the form of the web or the blanks of packaging material. Alternatively, or in combination, the packaging disinfection station 120 is configured to disinfect packages formed within the packaging machine 100. Especially, the packaging disinfection station 120 may be configured to disinfect packages formed within the packaging machine 100 before the respective package is filled with the liquid food product. More, specifically a packaging disinfection station 120 may be configured to disinfect an interior of packages formed within the packaging machine 100 before the respective package is filled with the liquid food product.

The packaging disinfection station 120 comprises a hydrogen peroxide supply 122, a spray nozzle 124 and a ionizer 126. The hydrogen peroxide supply 122 is configured to supply a liquid comprising hydrogen peroxide to the spray nozzle 124. The liquid comprises 3 -10 vol% of hydrogen peroxide. The rest of the liquid is typically water. The spray nozzle 124 is configured to distribute an aerosol of the liquid supplied by the hydrogen peroxide supply 122. The liquid supplied to the spray nozzle 124 is aerosolized using pressurized air. The spray nozzle 124 is configured to produce droplets of aerosol comprising hydrogen peroxide having a mean diameter of 8 - 50 micrometers. Accordingly, the spray nozzle 124 is configured to distribute an aerosol comprising hydrogen peroxide. The aerosol, just as the liquid, comprises 3 -10 vol% of hydrogen peroxide. The spray nozzle 124 is arranged such that the aerosol is distributed onto a surface of the packaging material or the packages.
The ionizer 126 is arranged in distance of 0,2 mm to 1 cm from an outlet of the spray nozzle 124 through which outlet the aerosol is ejected. The ionizer 126 is configured to ionize hydrogen peroxide of the aerosol before the aerosol is distributed onto the packaging material or onto the packages. The ionizer 126 may be a plasma generator comprising two pin electrodes between which a plasma is configured to be formed. The plasma is generated by continuously applying a voltage difference between the two pin electrodes. Such generated plasma is often referred to in the literature as a cold plasma. The aerosolized hydrogen peroxide may hence be activated, i.e. the hydrogen peroxide being ionized, by the cold plasma generated between two pin electrodes. According to one example, a distance between the two electrodes may be 9 mm and voltage applied between the two electrodes may be 17 kV. According to the same example a flow rate of the liquid supplied to the spray nozzle may be 5 mL/min with an air pressure of 7 psi (approximately 48.3 kPa). By applying a continues voltage difference between the two pin electrodes a continues formation of ionized hydrogen peroxide comprising hydroxyl radicals will be provided. Accordingly, the aerosol will comprise hydroxyl radicals and hence the aerosol distributed onto the packaging material or onto the packages will comprise the hydroxyl radicals.

The packaging machine may further comprise a removing station 130. The removing station 130 is configured to remove residue hydrogen peroxide from the disinfected packaging material or packages before the packages are filled with the liquid food product. The removing station 130 is removing the residue hydrogen peroxide with 90 °C to 200 °C hot HEPA filtered air.

The packaging machine may further comprise a filling station 140. The filling station 140 is configured to fill the packages to be filled with the liquid food product.

The packaging machine may further comprise a sealing station 150. The sealing station 150 is configured to seal packages filled with the liquid food product.

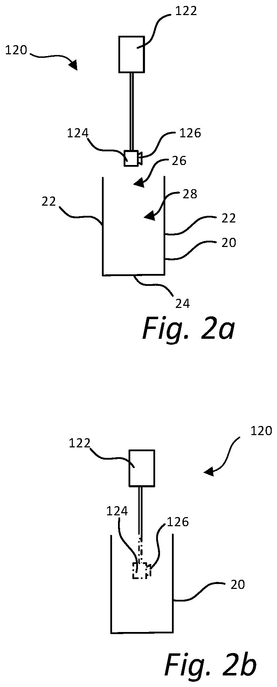

With reference to Figs 2a and 2b one exemplified embodiment of the packaging disinfection station 120 will be discussed. In Fig. 2a a package 20 to be disinfected is arranged in register with the packaging disinfection station 120 and in Fig. 2b the spray nozzle 124 (and the ionizer 126) of the packaging disinfection station 120 has been inserted into an interior 28 of the package 20. Inserting the spray nozzle 124 (and the ionizer 126) into the interior 28 of the package 20 may be made in different ways. For example, the package 20 may be moved relative to the spray nozzle 124 (and the ionizer 126), this is what is illustrated in Figs 2a and 2b. Alternatively, the spray nozzle 124 (and the ionizer 126) may be moved relative to the package 20. Yet alternatively, both the spray nozzle 124 (and the ionizer 126) and the package 20 may be moved.

The package 20 to be disinfected and later filled with liquid food product has a cross-section defined by a plurality of sidewalls 22, a closed end 24 engaging the sidewalls 22, and an open end 26. As discussed above, at least some of the package 20 consists of packaging material comprising a carton layer and at least one plastic layer. Typically, at least the sidewalls, and a bottom of the package 20 consists of packaging material comprising a carton layer and at least one plastic layer. The bottom of the final package may either be the closed end 24 or the open end 26 of the package 20 to be disinfected and later filled. That is, the package 20 to be disinfected and later filled may be either filled from the top or from the bottom of the final package. An example of a package that is filled from the bottom is Tetra Top^{®}. For Tetra Top^{®} the closed end 24 is a top portion of the final package, the closed end 24 of the Tetra Top^{®} is made from plastic material. An example of a package that is filled from the top is Tetra Rex^{®}. For Tetra Rex^{®} the closed end 24 is a bottom portion of the final package, the closed end 24 of the Tetra Rex^{®} is made from packaging material comprising a carton layer and at least one plastic layer.

Upon the spray nozzle 124 (and the ionizer 126) has been inserted into the interior of the package 20 to be disinfected, the spray nozzle 124 is configured to distribute an aerosol onto interior surfaces of the package 20 to be disinfected.

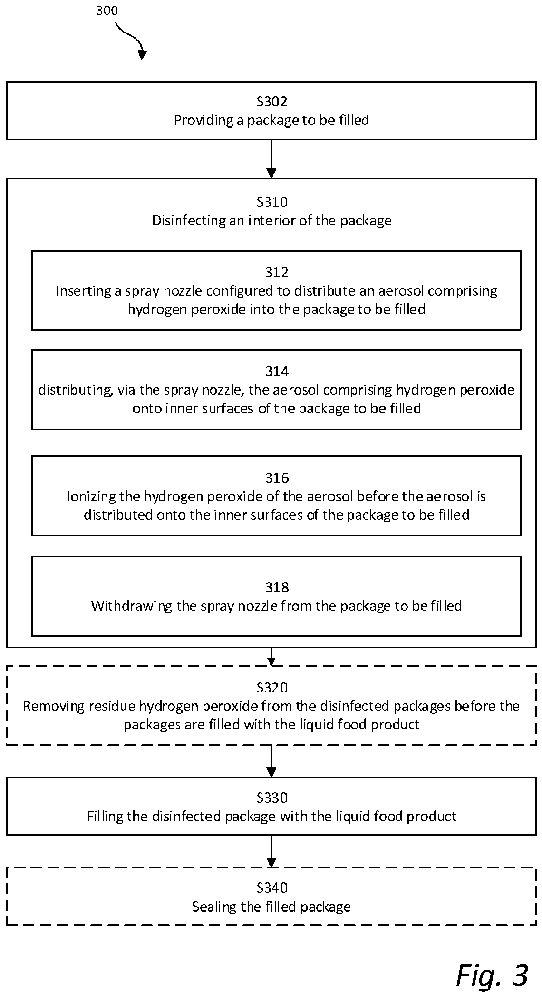

In connection with Fig. 3 a method 300 for filling a package with a liquid food product will be discussed. As discussed above, the packages may be carton based packages configured to be filled with food products. Below, the different steps of the method 300 are described in more detail. Even though illustrated in a specific order, the steps of the method 300 may be performed in any suitable order, in parallel, as well as multiple times. Typically the steps of the method 300 is performed using a packaging machine, e.g. the packaging machine 100 discussed in connection with Fig. 1. Some of the steps, or even all steps, of the method 300 may be executed by a control unit of the packaging machine. Some of the steps, or even all steps, of the method 300 may be computer implemented, i.e. some of the steps, or even all steps, of the method may be implemented as a computer code program being executed by a processor of the control unit of the packaging machine.

The method comprises providing S302 a package 20 to be filled, the provided package 20 to be filled having a cross-section defined by a plurality of sidewalls 22, a closed end 24 engaging the sidewalls 22, and an open end 26.

An interior 28 of the package 20 to be filled is then disinfected S310. The disinfection S310 is performed by: inserting S312 a spray nozzle configured to distribute an aerosol comprising hydrogen peroxide into the package 20; distributing S314, via the spray nozzle, the aerosol comprising hydrogen peroxide onto inner surfaces of the package 20; ionizing S316 the hydrogen peroxide of the aerosol before the aerosol is distributed onto the inner surfaces of the package 20; and withdrawing S318 the spray nozzle from the package 20.

After completed disinfection S310, filling S330 of the disinfected package 20 with the liquid food product is made.

The method may further comprising removing S320 residue hydrogen peroxide from the disinfected packages before the packages are filled S330 with the liquid food product. The removing may be performed with 90 °C to 200 °C hot HEPA filtered air.

The method may further comprise sealing S340 the filled packages. Especially, the open end 26 of the packages 20 is sealed so that a closed package is formed.

The person skilled in the art realizes that the present invention by no means is limited to what is explicitly described above. On the contrary, many modifications and variations are possible within the scope of the appended claims.

For example, during distribution of aerosol onto interior surfaces of a package 20, the spray nozzle 124 and/or the package 20 may be rotated. Alternatively, or in combination, the spray nozzle 124 may comprise a plurality of outlets (and ionizers 126) for distributing the aerosol.

Moreover, the packaging disinfection station 120 discussed in connection with Figs 2a and 2b is configured to be localized in the packaging machine 100 after one end of the package 20 is closed but before the package is filled and an open end of the packages is sealed. It is however, to be noted that according to another example, disinfection using ionized hydrogen peroxide in an aerosol distributed by the spray nozzle 124 may be made on the packaging material, especially on carton-based packaging material, before the actual packages are formed within the packaging machine 100.

Additionally, variations can be understood and effected by the skilled person in practicing the claimed invention, from a study of the drawings, the disclosure, and the appended claims.

## Claims

1. A packaging machine (100) configured to fill packages with a liquid food product, the packaging machine (100) comprising:
a feed unit (110) configured to feed packaging material to be formed to packages to be filled or to feed packages to be filled; and
a packaging disinfection station (120) configured to disinfect the packaging material or the packages, the packaging disinfection station (120) comprising:
a spray nozzle (124) configured to distribute an aerosol onto the packaging material or onto the packages,
a hydrogen peroxide supply (122) configured to supply a liquid comprising hydrogen peroxide to the spray nozzle (124); and
an ionizer (126) arranged in close proximity of an outlet of the spray nozzle (124) to ionize hydrogen peroxide of the aerosol before the aerosol is distributed onto the packaging material or onto the packages.

2. The packaging machine according to claim 1, wherein the ionizer (126) is a plasma generator comprising two pin electrodes between which a plasma is configured to be formed.

3. The packaging machine according to any one of claims 1-2, wherein the packages to be filled have a cross-section defined by a plurality of sidewalls, a closed end engaging the sidewalls, and an open end, wherein the spray nozzle (124) is configured to distribute the aerosol within an interior of the packages to be filled.

4. The packaging machine according to claim 3, wherein, at the packaging disinfection station (120), the spray nozzle (124) is configured to be inserted into the interior of the packages to be filled and upon the spray nozzle is inserted into the interior of a package to be filled the spray nozzle is configured to distribute the aerosol onto interior surfaces of the package to be filled.

5. The packaging machine according to any one of claims 1-4, wherein the aerosol comprises 3-10 vol% of hydrogen peroxide.

6. The packaging machine according to any one of claims 1-5, wherein the spray nozzle (124) is configured to produce droplets of aerosol comprising hydrogen peroxide having a mean diameter of 40 nanometers to 3 micrometers.

7. The packaging machine according to any one of claims 1-6, further comprising a removing station (130) configured to remove hydrogen peroxide from the disinfected packaging material or packages before the packages are filled with the liquid food product.

8. The packaging machine according to any one of claims 1-7, further comprising a filling station (140) configured to fill the packages to be filled with the liquid food product.

9. The packaging machine according to claim 8, further comprising a sealing station (150) configured to seal packages filled with the liquid food product.

10. The packaging machine according any one of claims 1-9, configured to fill at least 4500 packages per hour per packaging line.

11. A packaging disinfection station (120) configured to be feed with packaging material to be formed to packages (20) to be filled or to be feed with packages (20) to be filled, the packaging disinfection station (120) comprising:
a spray nozzle (124) configured to distribute an aerosol onto the packaging material or onto the packages (20);
a hydrogen peroxide supply (122) configured to supply a liquid comprising hydrogen peroxide to the spray nozzle (124);
an ionizer (126) arranged in close proximity of an outlet of the spray nozzle (124) to ionize hydrogen peroxide of the aerosol before the aerosol is distributed onto the packaging material or onto the packages (20).

12. The packaging disinfection station (120) according to claim 11,
wherein the packaging disinfection station is configured to be feed with packages (20) to be filled, the packages (20) to be filled having a cross-section defined by a plurality of sidewalls (22), a closed end (24) engaging the sidewalls, and an open end (26),
wherein the spray nozzle (124) is further configured to be inserted into an interior (28) of a package (20) to be filled and to distribute the aerosol onto interior surfaces of the package (20) to be filled.

13. The packaging disinfection station (120) according to claim 11 or 12, wherein the ionizer (126) is a plasma generator comprising two pin electrodes between which a plasma is configured to be formed.

14. A method for filling a package with a liquid food product, the method comprising:
providing (S302) a package to be filled, the provided package to be filled having a cross-section defined by a plurality of sidewalls, a closed end engaging the sidewalls, and an open end;
disinfecting (S310) an interior of the package to be filled by:
inserting (S312) a spray nozzle configured to distribute an aerosol comprising hydrogen peroxide into the package to be filled,
distributing (S314), via the spray nozzle, the aerosol comprising hydrogen peroxide onto inner surfaces of the package to be filled,
ionizing (S316) the hydrogen peroxide of the aerosol before the aerosol is distributed onto the inner surfaces of the package to be filled,
withdrawing (S318) the spray nozzle from the package to be filled;
filling (S330) the disinfected package with the liquid food product.

15. The method according to claim 14, further comprising removing (S320) residue hydrogen peroxide from the disinfected packages before the packages are filled with the liquid food product, wherein the removing is performed with 90 °C to 200 °C hot HEPA filtered air.
